# EUROPEAN PATENT SPECIFICATION

(11) **EP 0 773 717 B1**
(45) Date of publication and mention of the grant of the patent: **07.03.2001**
(21) Application number: 95922669.7
(22) Date of filing: 26.06.1995
(51) Int. Cl.: A01N 25/00, A61L 2/06

(54) **METHODS AND APPARATUS FOR DE-NATURING HOUSE DUST MITE (HDM) ALLERGEN**
VERFAHREN UND VORRICHTUNG ZUR DENATURIERUNG VON HAUSSTAUBMILBENALLERGEN
PROCEDE ET APPAREIL DESTINES A LA DENATURATION DE L'ALLERGENE DES ACARIENS DETRITICOLES

(30) Priority: 29.07.1994 GB 9415317; 20.09.1994 GB 9418933; 28.03.1995 GB 9506289
(43) Date of publication of application: 21.05.1997
(60) Divisional application: 00109341.8
(73) Proprietor: Houlbrook, Kenneth, Pateley Bridge HG3 5BN (GB)
(72) Inventor: Houlbrook, Kenneth, Pateley Bridge HG3 5BN (GB)
(74) Representative: Denmark, James
(86) International application number: GB9501498
(87) International publication number: WO9603870

(56) References cited:
- EP-A- 0 262 451
- EP-A- 0 395 787
- GB-A- 2 276 811
- GB-A- 2 280 851
- PATENT ABSTRACTS OF JAPAN vol. 15, no. 488 (C-0893) 11 December 1991 & JP,A,03 213 185 (PARAMAUNTO BED KK) 18 September 1991
- ALLERGY, vol.44, no.8, August 1989, COPENHAGEN (DK) pages 396 - 400 A.ANDERSEN ET AL 'House Dust Mite, Dermatophagoides pteronyssinus, and its allergens: effects of washing'
- PATENT ABSTRACTS OF JAPAN vol. 18, no. 501 (C-1251) 20 September 1994 & JP,A,06 169 862 (GOUNAI TOSHIKATSU) 21 June 1994
- J. ALLERGY AND CLINICAL IMMUNOLOGY, vol.81, no.4, April 1988, ST. LOUIS (US) pages 706 - 710 H.MOSBECH ET AL 'Control of House Dust Mites by Electrical Heating Blankets'
- ALLERGY, vol.49, no.2, February 1994, COPENHAGEN (DK) pages 131 - 133 H.S.M. KORT ET AL 'Four-year Stability of Der p I in House Dust under Simulated Domestic Conditions in vitro'

## Description

This invention relates to the denaturing of the HDM allergens, for the improvement of living conditions for asthma and the like sufferers, and the invention achieves this in various ways, involving methods, articles and equipment. The invention represents a breakthrough in relation to the problem that the HDM allergens are the major cause of discomfort to asthma and the like sufferers as evidenced by medical studies (See M J Bluff "Use of Liquid Nitrogen in the Control of House Dust Mite Population" Clinical Allergy, 1986 - volume 16, pages 41 to 47).

The present invention has particular application to the treatment of articles containing textiles, in particular voluminous soft furnishings such as mattresses, bed bases, pillows, sofas, cushions, soft toys and the like which constitute breeding grounds for the HDM. The treatment to which the invention relates is one whereby the HDM (Dermatophagoides Patheronyssinus) may be killed, and allergen contained in its droppings may be de-natured.

The HDM, as its name suggests, inhabits indoor environments, in particular those inhabited by humans or animals. Amounts of microscopic flakes of skin are shed continually by humans or animals, and these flakes fall to the floor and onto furnishings in a household, and where there is a fabric-covered article, the skin flakes will gradually seat within the article. The HDM feeds from these skin flakes as they decompose, and it will be appreciated that where large amounts of skin are collected, large concentrations of HDM are found. Textiles arc particularly susceptible to infestation by HDM since skin particles collect in the spaces between the fibres of the textile, in which spaces the HDMs are able to live and reproduce generally without excess hindrance. Bedding materials are particularly infested with HDMs since large amounts of skin are shed in beds, onto bed bases and duvets, and HDM concentrations build up unchecked.

Research has shown that the effects of living in environments free of HDMS, such as hospitals, where few places are found for the HDM to live and breed, is often beneficial to humans with a propensity to develop respiratory problems such as asthma and other diseases such as rinitis and eczema. It is believed that not the HDM itself, but an allergen or group of allergens called Der.pl (herein "HDM allergen"), associated with the faecal pellets of the mites, is a contributing factor if not a major cause of these diseases. It is believed that the HDM allergen causes an allergic reaction which brings about inflammation of tissue at the surface of the broncheoles in the lungs of an asthmatic person where the allergen comes to rest, where the inflammation causes breathing difficulties.

Various methods and means have been proposed to eliminate the HDM from bedding materials belonging to asthma sufferers in particular.

In the home there are several forms of textile article which form infestation grounds for the HDM and these are two-dimensional type sheet materials such as curtains, carpets, upholstery coverings and the like, which are difficult to clean, and three-dimensional textile articles such as pillows, cushions, mattresses, chairs, sofas, soft toys duvets and so on, and the present invention relates to the treatment of the so-called three-dimensional articles.

Some two-dimensional textile articles, such as clothes and bedclothes may be washed to eliminate the HDM and its allergen, but some of the articles containing a textile which are treated by the invention are obviously not susceptible to such treatment.

There have been attempts to provide treatment of and machines for treating textile articles for the elimination of the HDM and its allergens. There have also been proposed machines for the cleaning of the two-dimensional articles such as carpets by treatment which may involve the unwitting killing of the HDM and the denaturing of the allergens but the prior proposals have failed to provide a completely effective solution to the problem, by means of a quick and efficient treatment (which can be performed as a service to, or can be performed by a householder) which leaves the treated article completely or substantially free of HDM and denaturing the HDM allergens leaving the article in an HDM free condition after treatment.

Thus, there are known vacuum cleaners specially adapted for the removal of HDM from two-dimensional fabric Articles, the cleaners having powerful suction and fine filtering arrangements. In one specially adapted vacuum cleaner, once the dust has been vacuumed and collected, heat is applied to the collecting receptacle at 600C to kill the HDMs contained in the dust. These vacuum cleaners are not suitable for use in the cleaning of three-dimensional textile articles; the cleaner cannot apply the pressure required to effectively remove the embedded allergen particles, nor the large numbers of mites which remain in place by clinging onto the textile fibres. our research furthermore has shown that 60°C is an insufficient temperature to de-nature the allergen in a reasonable time.

A further product marketed for use in conjunction with the abovementioned vacuum cleaners is that of a pesticide spray which kills the HDM. Many people are however wary of using pesticide sprays, particularly in the household. Large amounts of the spray would be required in order to treat, for example, a single mattress. Furthermore, the spray may kill the HDMS, but the allergen is not affected and remains potent.

Another proposed method of preventing HDM from building up and the HDM allergen to become airborne is that of covering the bed with an air-impermeable sheet of plastic. However in general such plastic coverings have been found to reduce the levels of comfort achieved by the use of textile coverings only.

In British Patent Application No. 2280851, it has been proposed to use steam to de-nature the HDM allergen, but that patent application merely suggests the use of steam without giving further particulars for a complete and effective treatment. A drawing in the application shows what appears to be a steam generating machine and an outlet nozzle jetting steam into a floor covering, but no description is given in relation thereto. This patent application was not published until 15th February 1995.

British Patent No. 483710 discloses the same basic equipment for parasite diminution.
European Patent No. 0395781 Bl discloses a method of combating the HDM by means of a vacuum cleaner suction nozzle co-operating with a hot air source, which comprises a hot air jet at 60°C to 70°C directed to the textile material to be treated. Whilst this hot air may kill the HDM, it does not at least within a sensible period of time de-nature the HDM allergen. The patent in any event does not recognise the need to de-nature the HDM allergen and makes no reference thereto.

European Patent Application No. 0424070 Al describes one example of the use of a spray of treatment medium i.e. liquid nitrogen, for the killing of the dust mite; the application makes no disclosure concerning the denaturing of the HDM allergen.

British Patents Nos. 1286985, 1448434 and 1520761, and US Patent 3262146 disclose that carpets and the like may be cleaned by dry, super heated steam jets followed by vacuum application.

European patent application EP 262451 A describes a sterilisation method which comprises drying and heating moist objects whereby the temperature is raised slowly to 65°C and than more rapidly to 125-130°C. Japanese patent application JP 03213185 A describes a method of washing and sterilising mattresses which comprises applying detergent containing water on one side while applying a vacuum on the other side of the mattress followed by sterilisation and drying with vapour at high temperature and pressure. Japanese patent application JP 06169862 A describes a method to kill house dust mites by placing an article infested with house dust mites in a closed container followed by injecting a hot gas into the container. In Allergy 44, 396-400 (1989) it is taught that washing at 58°C removes house dust mites and significantly reduces the amount of allergenic proteins in the treated material but fails to reduce significantly the allergen activity. In Allergy 49, 131-133 (1994) it is taught that the house dust mite allergen Der.P1 is not de-natured, when house dust is heated at 60°C for 24 hours but that it is on the other hand known that it is de-natured to a great extent when heated at 56°C for 24 hours in solution and even more at 75°C for 1 hour.

There has been therefore much attention to the need to kill the HDM, but less attention to or realisation of the need to de-nature the HDM allergen and whilst this may take place in some of the prior art treatment by design or by accident, this realisation has contributed to the present invention.

This invention also recognises the need to provide a practical, quick and efficient treatment which can be carried out leaving the articles treated ready for use in as short a time as possible.

Our research has indicated that in preferred forms of the invention depending upon the treatment not only does heat need to be present to denature the HDM allergen, but moisture must also be present, if temperature and treatment time are to be kept to reasonable, practical levels. We believe the article must be at a sufficiently high temperature (80°-100°C) dependent upon time to de-nature the allergen and/or dry the article. In atmospheric conditions we have found that although the steam is jetted from a vessel in which it is contained at above atmospheric pressure, when it is released, the article cannot be heated to over 100°C. Fortunately, this is high enough to de-nature the HDM allergen and at the same time low enough to avoid damage to the article.

It is an object of the present invention, to provide a method of treating HDM containing articles whereby a large proportion of the HDKS are eliminated without the drawbacks and the inefficiencies of the known methods.

A further object of the invention is the killing of the HDM and by natural means which leave no harmful residues.

It is a yet further object of the invention to provide a method of destroying or denaturing insitu the HDM allergen which collects in the articles.

According to the present invention there is provided:- A method for the de-naturing of the HDM allergens in articles, such as pillows, cushions, mattresses, divans, duvets, chairs, settees, soft toys and the like of permeable textile materials, which are breeding grounds for the HDM, and which have a core, characterised by the step of injecting hot fluid into the core of the article so that it permeates the textile material from the core outwardly to de-nature the HDM allergen.

The article should be heated to a sufficient temperature and for a sufficient time in order to kill a substantial proportion of the HDMs. Preferably, the article or material is heated to at least 60°C. The time required will reduce as the temperature achieved increases.

Many soft furnishing articles contain fabric of synthetic materials such as nylon, and it will often be essential that the article is not irreparably damaged during the treatment. It has been estimated that a maximum "safe" final temperature for the article undergoing treatment may be up to 120°C or sometimes even 150°C, in order to obviate damage to the least heat-resistant materials.

An optimum temperature may be of the order of 100°C, which if maintained over a period of say ten minutes will ensure that the majority of HDMs are killed and the majority of the allergen material will be de-natured, whilst avoiding harm to heat sensitive textile materials.

Steam will preferably be used, which supplies both moisture and heat to the fibrous material. Steaming the article or material to become moist during treatment and a forced drying after the moisture treatment is an important step, as the treatment can be effected quickly and effectively. The article might also suitably be force heated before steaming by the application of heated air.

No prior art method has suggested heating from the core to ensure that effective heating of the article throughout its thickness takes place and when the temperature of the article at its surface reaches the desired temperature, effective heating has been completed.

Heated fluid may be injected into the core of the article to heat through the fabric covering of the article by the use of an applicator head connected to a hot gas blower, the head having a lance which projects into the article.

When a heating apparatus uses a steam heat exchanger to provide hot air by heat exchange between the heat exchanger coils through which steam passes, such apparatus is particularly adaptable for the present invention, in that the steam circuit can be provided with a valve for the selective bleeding of steam therefrom, whereby there is a supply of hot air on the one hand and a supply of steam on the other hand which can be used selectively for applying moisture and heat (steam supply) and hot air on the other hand f or the forced drying step as indicated above.

Such an apparatus may be portable and comprise outlets for steam, and hot air, and optionally vacuum, connected by appropriate piping, for example to a generating vehicle provided with a steam generator, blower and vacuum generator.

Where the article, as in the case of conventionally sprung mattresses, contains a sizable internal cavity surrounded by a textile covering, the cavity may be utilized in order to distribute the heated fluid throughout the interior of the article, and the applicator conduit may be used to transmit/ the heated fluid directly into the cavity to enable a full permeation of the heated fluid through the textile. The applicator conduit may be in the form of an apertured conduit located to lie along the length of the mattress, the apertures allowing release of the heated fluid at regular intervals 'along its length.

Even when the article has no large internal cavity, for example as in the case of a pillow, seat or cushion, homogenous permeation of heated fluid throughout the article using a single lance may still be affected. If the article has multiple cavities, in order to heat the fabric, a number of applicator conduits or lances may be inserted to lie with outlets distributed regularly throughout the article and/or textile. Each such conduit preferably comprises a number of outlets along its length internally of the article.

The article may be externally insulated during the permeation of the heated fluid in order to reduce the energy and time required in order to effect treatment, thereby making the process more efficient. The article may suitably be wrapped in an insulating foam rubber covering. The covering may be specially adapted to receive the article snugly, with the covering having an aperture allowing the insertion of the applicator lance or lances.

When so insulated, and pressurised heating fluid is delivered to the article to heat same, the fluid may be recirculated to conserve energy and promote the permeation of the heated fluid throughout the fabric of the article.

The invention also provides an article such as a pillow, cushion, mattress, divan, duvet, chair, settee, soft toy and the like of permeable textile material, which is a breeding ground for the HDM and which has a core, characterised in that the article is adapted to be treated by being heated, by the injection of hot fluid medium so that it permeates the textile material from the core outwards, for the de-naturing, at periodic intervals, of the HDM allergen, said adaptation being characterised in that the article is provided with an integral coupling means for coupling to a portable hot fluid injection device by which the fluid which performs the de-naturing can be injected into the core of the article so as to permeate through the article from the core to the outside thereof.

Also, according to the invention there is provided an article such as a pillow, cushion, mattress, divan, duvet, chair, settee and the like of permeable textile material, which is a breeding ground for the HDM and which article has a core, characterised in that the article is adapted to be treated by being heated, by causing hot fluid medium to flow from the core so that it will permeate from the core outwards to the surface of the article, for the de-naturing, at periodic intervals, of the HDM allergen in the textile material, said adaptation comprising providing the article with an integral hot air blower which can be switched on and off.

The invention also provides an apparatus for the de-naturing of the HDM allergens in three-dimensional articles such as pillows, curtains, mattresses, divans, duvets, chairs, settees, and the like which are of permeable textile materials which are breeding grounds for the HDM comprising means for producing a flow of heated fluid and characterised by a lance adapted to be penetrated into the core of the article for the release of heated fluid in the core of the article so that it can heat the article by permeating from the core to the outside of the article, and characterised in that the lance is designed to have a first lance tube by which steam can be injected into the article core and a second lance tube by which hot air can be injected into the article core, and wherein the first lance tube is located inside the second lance tube.

Embodiments of the invention will now be described, by way of example, with reference to the diagrammatic drawings, wherein:-
Fig. 1 is an enlarged view showing a method of treatment according to the invention, the article being treated being a mattress;
Fig. 2 is a view similar to Fig. 5 but showing the treatment as applied to a pillow;
Fig. 3 is a perspective view of a pillow showing an article according to the invention;
Fig. 4 is a further enlarged view of an injection lance suitable for use in the methods of the invention shown in Figs. 1 and 2.
Fig. 5 is a plan view of an equipment usable in the methods shown in Figs. 1 and 2; and

The present invention is concerned with the treatment of articles which form breeding grounds for the HDM. The invention addresses the need on the one hand to eliminate the HDM, and on the other hand to de-nature the HDM allergens. In the invention, attention is given to the de-naturing of the HDM and the preferred method involves the use of moisture, preferably hot water or steam, because according to our research work, it has been discovered, at least at the temperatures used in the tests, that moisture must be present for the effective denaturing of these allergens. Minimally the invention involves the application of heat, and at the temperatures which we were able to achieve in practise for heating the articles and using steam, the presence of moisture is necessary to de-nature the allergens. In practise using steam, tests showed that it was difficult to heat any article to a temperature above 100°C.

In the invention, heat is used to provide an advantageous effect. It is of course preferred that water be used for the effective denaturing of the allergens, but the invention is not limited in this way. The invention also concerns articles which are specifically adapted to enable treatment to be effected thereon. The concept is that these articles will be used in place of the conventional articles such as pillows and mattresses, but will be so designed that they can readily be treated simply by bringing the treatment equipment to the location of the article and by coupling it to the article by the special adaptation. For example the bedroom of an asthma sufferer may be provided with a specially adapted mattress and pillow which can be heated from the core at will by the coupling of injection apparatus for injecting heating fluid into the core of the mattress and pillow. If this is done on a regular basis, the effective HDM removal and HDM allergen denaturing will take place.

The invention further provides specifically adapted apparatus which is particularly effective in specific applications.

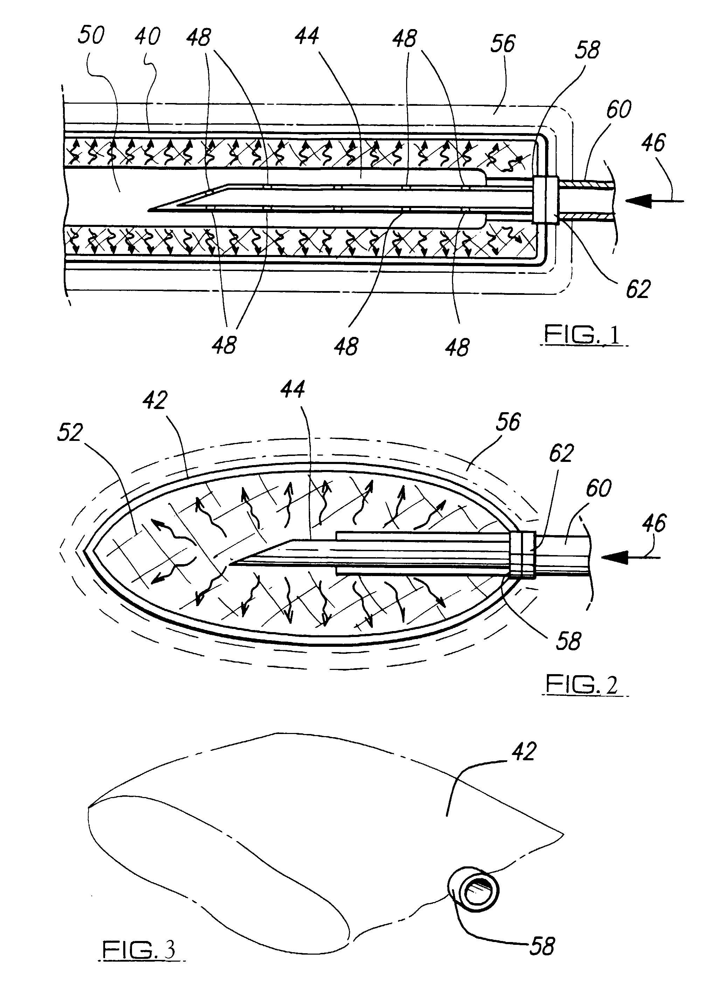

Figs. 1 and 2 illustrate embodiments of the invention. In Fig. 1 a mattress to be treated is indicated by reference 40, whilst reference 42 in Fig. 2 illustrates a pillow to be treated. In each case, an injection lance 44 projects into the core of the article, and hot fluid is injected into the lance as indicated by arrow 46 so as to heat the article from the core. The hot fluid injected into the lance 46 issues from the lance inside the article through outlets 48 and the hot fluid permeates from the core to the outer surface of the article, heating and treating same as it thus permeates.
In the case of the mattress of Fig. 1, it is shown with a hollow interior 50, but in the case of the pillow, it is shown as being filled with pillow filling 52.

Also shown in Figs. 1 and 2 is the provision of a heat retaining covering 56 which is wrapped around the article as it is treated, in order to retain the heat. The pillow and mattress is each provided with a special coupling 58 by which it can be readily coupled to the lance supply pipe 60, which is provided with a co-operating coupling 62. In use therefore it is simply a matter of bringing the lance 44 to the article and by pushing it into the core of the article followed by establishment of the coupling 58/62 which may be of the quick release variety. The hot fluid is then injected into the interior of the article to treat same. In a preferred method, hot air is first injected to pre-heat the article, and then steam is injected for the heating and moistening of the article, followed by the injection of hot air.

The approach of providing heating from the core of the article has not previously been suggested in the prior art, and therefore this invention has various different embodiments. For example, instead of using steam it may be possible to achieve an appropriate effect by the use of hot air only.

Additionally, the hot air may be provided by providing the article with an integral internal hot air blower which can be switched on selectively. Furthermore, the interior of the article may additionally be provided with a heating element similar to an electric blanket.

Fig. 3 shows the pillow of Fig. 6 in perspective view, and the coupling 58 which is integral with the pillow is clearly shown. An article provided with this special adaptation is of particular advantage, and constitutes a third aspect of the invention, because it is envisaged that in the provision of the service of denaturing HDM allergen and eliminating the HDM will involve the provision of not only the equipment for effecting the treatment, but also the specially adapted articles to be used by sufferers. An asthma sufferer therefore may have his bed, pillows, cushions, seats, sofas provided with special adaptations and constructions to enable a contractor to come and to treat the articles for achieving the appropriate effect as described herein. Indeed, it may be possible to provide each sufferer with his own treatment equipment so that he can perform the treatment on his or her own.

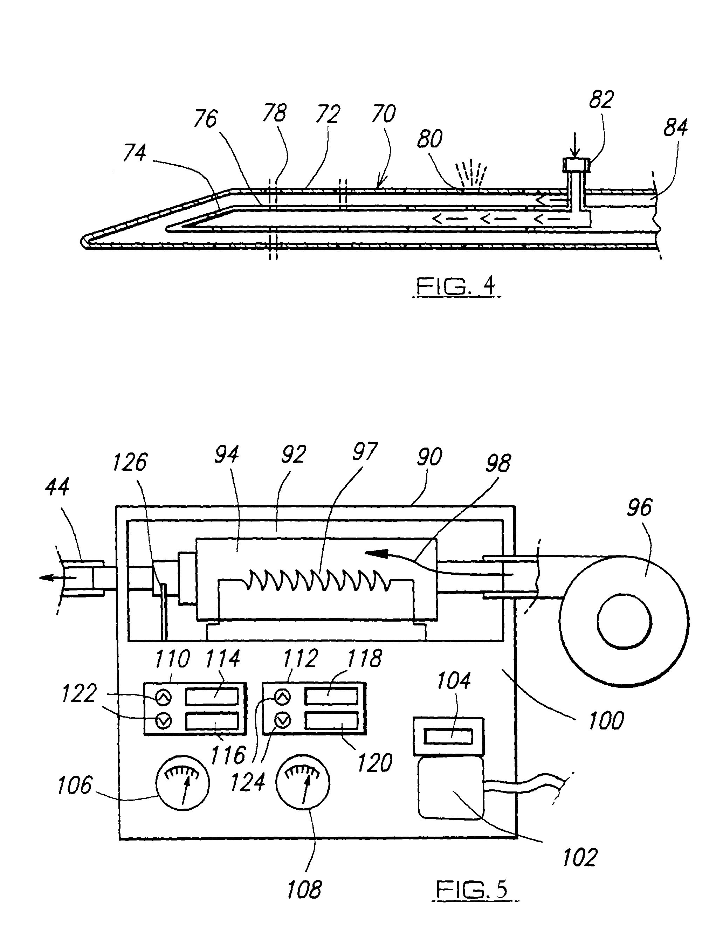

Fig. 4 shows that a specially adapted lance may be used for the injection of the steam and the hot air rather than using separate lances. In the arrangement of Fig. 4, the lance 70 comprises an outer tube 72 from which the hot air issues, and an inner tube 74 from which the steam issues. The inner tube 74 has small jetting apertures 76 from which the steam issues as indicated by reference 78, and these apertures are in alignment with larger air outlets 80 in the tube 72. The steam inlet 82 is connected to the inner tube, whilst the air inlet is connected at region 84 to the hot air generator as will be explained in relation to Fig. 5.

Referring now to Fig. 5. this figure illustrates diagrammatically an equipment for the injection of hot air into an article as shown in Fig. 1 or Fig. 2.

The equipment comprises a casing 90 which typically would be of a size capable of being carried by one man. The casing has a cavity 92 in which is contained a process heater 94 for heating the air which is driven therethrough by means of a blower 96. This blower may be detachable for transportation. The cold air blown by the blower 96 passes as indicated by arrow 98 over the resistance heater 97 contained in the heater 94, to be heated thereby, and is driven into the lance 44 and eventually is distributed throughout the article as shown in Fig. 1 or Fig. 2.

Reference numeral 100 represents the portion of the casing which contains the controls, and an electrical supply is connected thereto as indicated by reference 102, which is controlled by switch 104. Dial 106 is an ammeter to show the current being drawn, whilst dial 108 shows a pressure gauge which indicates that the pressure of the air being supplied to the interior of the article.

The sub panels 110 and 112 represent indicators for selected temperature in display 114 and actual temperature in display 116 on the one hand, and selected time display 118 and actual time display 120 on the other hand. These units have up and down setting buttons 122 and 124 by which the set temperature and set time in displays 114 and 118 can be adjusted.

When the system is running, the temperature of the air sensed by thermocouple 126 will be displayed in display 116, and the elapsed time of the treatment will be indicated in display 120.

The equipment is controlled insofar as if there is a loss of pressure in the process heater 94, the heating element 97 is automatically switched off so that damage thereto will be avoided.

The time of each treatment will be varied, but it has been indicated that in the order of ten minutes at least will be required for the steaming of the article, when steam is supplied, and ten minutes heating will be required in order to dry off the moisture left from the steam treatment.

The process heater may have a power rating in the order of 5,500 watts for use with double bed mattresses, although a lower rating, perhaps in the order of 3,000 watts may be suitable for the mattresses for a single bed.

Instead of the lance being provided with a coupling and the article being adapted for receipt of the lance, the lance may simply be used to puncture an existing conventional article to be treated, and the puncture duly closed and repaired following treatment.

Where the article is encased in insulation, that insulation may cover the article fully except for in an area to receive the lance, and the covering may comprise a heating element for applying heat to the outer surface of the article.

The heating of the article is in effect causing all of the article to be raised to elevated temperature for the effective treatment thereof.

Using hot air it is possible to heat the article to a temperature such that the outer surface of the article reaches 120°C. This temperature was maintained for 5 to 10 minutes.

The insulating sheets may be air permeable and air outlet apertures may be provided in the sheath to promote the permeation of the heated air through the article being treated. On the other hand, the sheath may be air impermeable but be provided with an air outlet to allow the air to escape through such outlet, and such outlet can be coupled to the blower in order that the heated air will be re-circulated.

The heating may be thermostatically controlled by known methods in addition to or in place of the pre-setting of the temperature as described in relation to the apparatus in Fig. 5.

The sheathing of the article being heated may be varied in order to achieve best flow of the heating fluid therethrough. Thus, the size of a mattress may be sealed by means of sealing tape.

Instead of there being one lance injected into the article to be treated, where appropriate, there may be several of said lances coupled to a common source of supply of hot fluid.

A major practical application of the invention is the effective treatment of mattresses; no prior method has effectively addressed the difficulty of effectively treating mattresses for the elimination of the HDM and the denaturing of the HDM allergens. All prior treatments have been surface treatment suggestions which are unlikely to be effective in failing to give sufficient penetration for treating the deeper lying layers.

A mattress may consist of a steel frame, resilient steel springs, textile paddings respectively wrapped over on the upper and lower regions of the frame but not extending to an intermediate region around the periphery of the mattress, and an outer fabric or textile covering. Interiorly of the sprung frame may be an air-filled cavity. The textile paddings of a mattress commonly consist of various layers to increase the comfort of the mattresses, often including layers of horse-hair matting and layers of synthetic material such as nylon. All of these textile layers are liable to become infested with HDM through time, in particular those on the upper padding where the largest quantities of shed skin collect since it is on the upper surface that a person sleeps, continually shedding skin fragments which penetrate deeper and deeper into the upper mattress padding, and then onto the lower padding, and the skin particles may eventually penetrate the lower padding and become deposited onto the bed base. Thus, both mattresses and bed bases are potential sources of the HDM allergen.

It is to be mentioned that the use of an insulating sheath 22 may not be necessary if a sufficient temperature can be achieved without the sheath. It may also be possible to achieve the required temperatures for example by the use of an insulating substantially air-impervious insulating strip surrounding the sides of the mattress, from which most of the hot air would otherwise escape rather than permeating through the padded fabric regions. This insulating strip would allow an aperture similar to aperture 24 in sheath 22 to provide access for the air blower head 20 to the side of the mattress cover 14. Whichever means of insulating is used, if indeed any at all is used , it is preferred that all of the mattress fabrics, including the paddings 8 and 10 and the cover 14, can be effectively elevated in temperature in order to substantially eradicate the HDM and its allergen from those fabrics in a single treatment of the mattress. In order to de-nature the HDM allergen moisture, preferably in the form of steam, should be supplied in combination with heated air or alone, and throughout the mattress materials.

By adopting an arrangement whereby the hot gases are effectively distributed throughout the fabric-containing portions of the article from the core thereof, the required temperature conditions can be achieved throughout the fabrics of the mattress. As the heated gas or gases permeates throughout the body of the pocket-sprung mattress it moistures and elevates the temperature thereof sufficiently to kill and the HDM/de-nature the HDM allergen as required.

In order to kill the HDM the temperature of the fabric and the gas permeating through the fabric must be sufficiently elevated for a sufficient period of time. Furthermore, in order to de-nature the HDM allergen, sufficient moisture must be supplied in combination with the heat, and to provide an effective service, the article must be force dried if steam is used. If a relatively low elevated temperature is used, the elevated temperature must be maintained over a longer period of time than that required at higher temperatures.

In a series of experiments which we have carried out, reductions in levels of the HDM allergen (Der.pl) implanted in a mattress were measured after various treatments with steam and hot air. The hot air and steam were injected directly into the body of the mattress by means of a 3.5 kW process heater and a 3 kW domestic steamer respectively.

In the first experiment, steam alone was injected over a period of 20 minutes. In the second, a hot air and steam combination and then hot air only was injected each for 10 minutes respectively. In the third, steam and hot air were supplied consecutively over a period of 15 minutes. The fourth experiment involved hot air alone, followed by steam alone, and followed by hot air alone f or a period of five minutes each. In the fifth experiment, hot air and steam were supplied in combination for a period of 15 minutes. Finally, the HDM allergen was incubated in a dry state in a hot air oven at 120° for 15 minutes.

It was found that reductions of generally at least 90%, and in one case, 99.9%, were obtained in the levels of allergen at the test sites in the mattress in respect of the first five experimental methods all involving steam. In contrast, the dry treatment in the hot air oven resulted in no measurable decrease in the levels of HDM allergen over the unheated control sample.

The treatment of a mattress or other article containing fabric may be more time consuming than these indicated times, since additional time should be allowed for elevating the temperature throughout the materials of the article to the required temperature. The use of permeating hot gas ensures that this additional time requirement may be kept to a minimum and the effectiveness of the process is maximised so as to readily destroy the HDM and its allergen throughout the fabric materials.

Where the article is large and well insulated, the article may be injected with gas initially at one rate of calorific delivery for a first period of time in order to heat the article to the desired temperature, and subsequently the gas may be injected at a lower calorific rate in order to maintain the desired temperature. This procedure ensures that treatment may be achieved in as little time as possible whilst conserving energy.

The invention provides an effective method and means for the denaturing of HDM allergens, and the various steps employed also address the matter of extermination of the HDM itself although, as indicated herein, it is the HDM allergens which are the major cause of discomfort in persons with respiratory problems.

Of particular significance is the utilisation of heat in conjunction with moisture either in the form of water or steam for the denaturing of the allergens as it has been found that it is necessary to use moisture to make the denaturing effective as indicated by the tests described herein.

In practise, because the steam is applied mainly to articles, when the steam is applied to the material or article it will be at atmospheric pressure, which in turn means that in practise it will not be capable of heating the article using the steam to more than 100°C, but fortunately, such a temperature is adequate to de-nature the allergen and kill the HDM. In the invention, it is preferred that the article be heated to a temperature in the order of 100°C. If the whole article is to be heated from its core up to a temperature of 100°, this may require the use of an insulating sheathing to prevent heat loss, but there will be effective and complete denaturing of the allergens and extermination of the house dust mite.

It can be seen therefore that the invention provides a means whereby, for example, a servicing company can visit domestic dwellings and other locations in order to perform the denaturing and elimination of HDM allergen and HDM. Such a service can be carried out on a periodic basis providing for example asthma sufferers with a continuing HDM and HDM allergen free environment, which will be extremely important to and provide great relief for asthma sufferers.

The process is made particularly effective if the articles to be treated in this way are custom designed so as to have an integral coupling and conduit means leading to the core of the article, and such coupling is designed to intercouple with an outlet of portable equipment which the contractor who will perform the service brings with him when he comes to treat the articles in a household.

The invention therefore also provides a combined set of equipment and articles to be treated, the articles to be treated being articles of domestic use such as pillows and mattresses which remain in situ in each customer's dwelling, whereas the portable apparatus which is designed to intercouple with the articles is the unit which is retained by the contractor. It can be seen therefore that a network of equipment located throughout the country can be used for performing a denaturing service an articles in domestic dwellings. Indeed, in some cases it may be appropriate to design and produce a set of equipment which is for retention in a household so that the owner can perform the denaturing and HDM elimination on his own. An excellent new and advantageous service is created by the invention which will provide extended benefit to people with respiratory problems.

Our work has shown that when moisture is used for the denaturing and HDM elimination process, the use of drying of the residual moisture enhances the operation, and is particularly convenient in enabling the process to be completed as soon as possible. It is desirable therefore that after the moisture treatment, the article or material is dried using heated gas, and in particular hot air. The hot air preferably is injected into the core of the article (although other methods are possible as described herein) and the air, like the steam before it heats the article from the core, and dries off any residual moisture. The air like the steam moves outwardly through the article to the surface thereof..

The invention provides a solution to a problem which is in fact endemic in the United Kingdom and is on the increase. The problem also exists in other countries throughout the world.

## Claims

1. A method for the de-naturing of the HDM allergens in articles, such as pillows, cushions, mattresses, divans, duvets, chairs, settees, soft toys and the like of permeable textile materials, which are breeding grounds for the HDM, and which have a core, characterised by the step of injecting hot fluid into the core of the article so that it permeates the textile material from the core outwardly to de-nature the HDM allergen.

2. A method according to claim 1, characterised in that the hot fluid includes steam.

3. A method according to claim 1 or 2, characterised in that the hot fluid includes hot air.

4. A method according to any of claims 1, 2 or 3, characterised in that the hot fluid includes hot air supplied by means of a blower of hot air embodied in the core of the article.

5. A method according to any of claims 1 to 4, characterised by the step of covering the article by heat insulation means during the permeation of the hot fluid.

6. A method according to any preceding claim, characterised in that the hot fluid which passes to the surface of the article is re-circulated by being returned to the core and heat is added thereto as it is re-circulated.

7. The method according to any of claims 1 to 6, characterised in that the article core has one or more cavities into which the hot fluid is injected.

8. The method according to any preceding claim, characterised in that steam is initially injected into the core, and subsequently hot air is injected into the core to remove any residual moisture left by the steam injection.

9. The method according to any preceding claim, wherein the hot fluid comprises hot air which is injected into the core to preheat same, and steam injected subsequent to the hot air.

10. An article such as a pillow, cushion, mattress, divan, duvet, chair, settee, soft toy and the like of permeable textile material, which is a breeding ground for the HDM and which has a core, characterised in that the article is adapted to be treated by being heated, by the injection of hot fluid medium so that it permeates the textile material from the core outwards, for the de-naturing, at periodic intervals, of the HDM allergen, said adaptation being characterised in that the article is provided with an integral coupling means for coupling to a portable hot fluid injection device by which the fluid which performs the de-naturing can be injected into the core of the article so as to permeate through the article from the core to the outside thereof.

11. An article according to claim 10, characterised in that the article has a hollow interior.

12. An article according to claim 11, characterised in that the hollow interior comprises a single cavity or a plurality of cavities.

13. An article such as a pillow, cushion, mattress, divan, duvet, chair, settee and the like of permeable textile material, which is a breeding ground for the HDM and which article has a core, characterised in that the article is adapted to be treated by being heated by causing hot fluid medium to flow from the core so that it will permeate from the core outwards to the surface of the article, for the de-naturing, at periodic intervals, of the HDM allergen in the textile material, said adaptation comprising providing the article with an integral internal hot air blower which can be switched on and off.

14. An apparatus for the de-naturing of the HDM allergens in three-dimensional articles such as pillows, curtains, mattresses, divans, duvets, chairs, settees, and the like which are of permeable textile materials which are breeding grounds for the HDM comprising means for producing a flow of heated fluid and characterised by a lance adapted to be penetrated into the core of the article for the release of heated fluid in the core of the article so that it can heat the article by permeating from the core to the outside of the article, and characterised in that the lance is designed to have a first lance tube by which steam can be injected into the article core and a second lance tube by which hot air can be injected into the article core, and wherein the first lance tube is located inside the second lance tube.

15. Apparatus according to claim 14 characterised in that said means for providing a flow of heated fluid comprises a process heater element, and an arrangement for causing the fluid to flow under pressure over the heater element on its way to the lance.

16. Apparatus according to claim 15, including a pressure responsive switch operative to cut out the heater element in the event that the pressure of the fluid passing through the heater element drops.

17. Apparatus for use in carrying out the method of claim 1, characterised by a steam driven heat exchanger, means for passing air through the heat exchanger to be heated thereby and so provide the means for force drying the material or article, and controlled bleed means enabling the bleeding of said steam to provide the means for rendering the material or article moist.

## Patentansprüche

1. Verfahren zur Denaturierung von Hausstaubmilben-Allergenen in Gegenständen wie Kissen, Polstern, Matratzen, Liegen, Steppdecken, Stühlen, Sofas, Stofftieren und dergleichen aus durchlässigen Textilmaterialien, die für Hausstaubmilben eine Brutstätte bilden und einen Kern haben, gekennzeichnet durch den Schritt des Injizierens von heißem Fluid in den Kern des Gegenstands in einer solchen Weise, dass es das Textilmaterial vom Kern nach außen hin durchdringt, um das Hausstaubmilben-Allergen zu denaturieren.

2. Verfahren nach Anspruch 1, dadurch gekennzeichnet, dass das heiße Fluid Dampf enthält.

3. Verfahren nach Anspruch 1 oder 2, dadurch gekennzeichnet, dass das heiße Fluid heiße Luft enthält.

4. Verfahren nach einem der Ansprüche 1, 2 oder 3, dadurch gekennzeichnet, dass das heiße Fluid heiße Luft enthält, die mit Hilfe eines Heißluftgebläses zugeführt wird, das im Kern des Gegenstands enthalten ist.

5. Verfahren nach einem der Ansprüche 1 bis 4, gekennzeichnet durch den Schritt des Abdeckens des Gegenstands mit einem Wärmedämmmittel während des Durchdringens des heißen Fluids.

6. Verfahren nach einem der vorherigen Ansprüche, dadurch gekennzeichnet, dass das heiße Fluid, das zur Oberfläche des Gegenstands strömt, rezirkuliert wird, indem es zum Kern zurückgeführt wird, und während seiner Rezirkulation Wärme hinzugefügt wird.

7. Verfahren nach einem der Ansprüche 1 bis 6, dadurch gekennzeichnet, dass der Kern des Gegenstands einen oder mehrere Hohlräume hat, in den/die das heiße Fluid injiziert wird.

8. Verfahren nach einem der vorherigen Ansprüche, dadurch gekennzeichnet, dass zuerst Dampf in den Kern injiziert wird und anschließend heiße Luft in den Kern injiziert wird, um durch die Dampfinjektion hinterlassene Restfeuchtigkeit zu entfernen.

9. Verfahren nach einem der vorherigen Ansprüche, bei dem das heiße Fluid folgendes umfasst: heiße Luft, die in den Kern injiziert wird, um diesen vorzuwärmen, und Dampf, der im Anschluss an die heiße Luft injiziert wird.

10. Gegenstand wie z.B. ein Kissen, ein Polster, eine Matratze, eine Liege, eine Steppdecke, ein Stuhl, ein Sofa, ein Stofftier und dergleichen aus durchlässigem Textilmaterial, der eine Brutstätte für Hausstaubmilben bildet und einen Kern hat, dadurch gekennzeichnet, dass der Gegenstand so ausgestaltet ist, dass er durch Erwärmen behandelt werden kann, indem heißes Fluidmedium auf eine solche Weise injiziert wird, dass es das Textilmaterial von dem Kern nach außen hin durchdringt, um das Hausstaubmilben-Allergen in regelmäßigen Abständen zu denaturieren, wobei die genannte Ausgestaltung dadurch gekennzeichnet ist, dass der Gegenstand ein integriertes Verbindungsmittel zum Verbinden mit einer tragbaren Heißfluidinjektionsvorrichtung aufweist, mit der das Fluid, das die Denaturierung bewirkt, in den Kern des Gegenstands injiziert werden kann, um den Gegenstand von seinem Kern zu seiner Außenseite zu durchdringen.

11. Gegenstand nach Anspruch 10, dadurch gekennzeichnet, dass der Gegenstand ein hohles Inneres hat.

12. Gegenstand nach Anspruch 11, dadurch gekennzeichnet, dass das hohle Innere einen einzelnen Hohlraum oder eine Mehrzahl von Hohlräumen umfasst.

13. Gegenstand wie z.B. ein Kissen, ein Polster, eine Matratze, eine Liege, eine Steppdecke, ein Stuhl, ein Sofa und dergleichen aus durchlässigem Textilmaterial, der eine Brutstätte für Hausstaubmilben bildet und einen Kern hat, dadurch gekennzeichnet, dass der Gegenstand so ausgestaltet ist, dass er durch Erwärmen behandelt werden kann, indem heißes Fluidmedium so von dem Kern strömen gelassen wird, dass es den Gegenstand vom Kern nach außen hin zur Oberfläche des Gegenstands durchdringt, um das Hausstaubmilben-Allergen im Textilmaterial in regelmäßigen Abständen zu denaturieren, wobei die genannte Ausgestaltung das Ausstatten des Gegenstands mit einem integrierten Innenheißluftgebläse umfasst, das ein- und ausgeschaltet werden kann.

14. Vorrichtung zum Denaturieren von Hausstaubmilben-Allergenen in dreidimensionalen Gegenständen wie z.B. Kissen, Vorhänge, Matratzen, Liegen, Steppdecken, Stühle, Sofas und dergleichen aus durchlässigen Textilmaterialien, die für Hausstaubmilben eine Brutstätte bilden, umfassend ein Mittel zum Erzeugen eines Heißfluidstroms und gekennzeichnet durch eine Lanze, die so ausgestaltet ist, dass sie in den Kern des Gegenstand eingeführt werden kann, um erhitztes Fluid im Kern des Gegenstands freizusetzen, so dass es den Gegenstand erwärmen kann, indem es ihn von seinem Kern zu seiner Außenseite durchdringt, und dadurch gekennzeichnet, dass die Lanze eine erste Lanzenröhre aufweist, durch die Dampf in den Kern des Gegenstands injiziert werden kann, sowie eine zweite Lanzenröhre, durch die heiße Luft in den Kern des Gegenstands injiziert werden kann, wobei sich die erste Lanzenröhre innerhalb der zweiten Lanzenröhre befindet.

15. Vorrichtung nach Anspruch 14, dadurch gekennzeichnet, dass das genannte Mittel zum Bereitstellen eines Heißfluidstroms ein Prozessheizelement und eine Anordnung umfasst, die das Fluid unter Druck über das Heizelement auf seinem Weg zur Lanze strömen lässt.

16. Vorrichtung nach Anspruch 15, umfassend einen auf Druck ansprechenden Schalter, der die Aufgabe hat, das Heizelement in dem Fall, dass der Druck des durch das Heizelement strömenden Fluids fällt, abzuschalten.

17. Vorrichtung zur Verwendung bei der Durchführung des Verfahrens nach Anspruch 1, gekennzeichnet durch einen dampfgetriebenen Wärmetauscher, ein Mittel zum Leiten von Luft durch den Wärmetauscher, die dadurch erwärmt werden soll, um auf diese Weise das Mittel zum Zwangstrocknen des Materials oder Gegenstands bereitzustellen, und ein gesteuertes Entlüftungsmittel zum Ablassen des genannten Dampfs, um das Mittel zum Befeuchten des Materials oder Gegenstands bereitzustellen.

## Revendications

1. Procédé destiné à la dénaturation des allergènes des acariens détriticoles (HDM) dans des articles comme les oreillers, les coussins, les matelas, les sofas, les couettes, les fauteuils, les canapés, les jouets en peluche et autres réalisés en matières textiles perméables qui constituent des milieux de reproduction favorables pour les HDM, et qui possèdent un embout caractérisé par l'étape consistant à injecter du liquide chaud dans le noyau de l'article, de sorte qu'il imprègne le matériau textile à partir du noyau jusque vers l'extérieur afin de dénaturer l'allergène des HDM.

2. Procédé, selon la revendication 1, caractérisé en ce que le liquide chaud comprend de la vapeur.

3. Procédé, selon la revendication 1 ou 2, caractérisé en ce que le liquide chaud comprend de l'air chaud.

4. Procédé, selon l'une quelconque des revendications 1, 2 ou 3, caractérisé en ce que le liquide chaud comprend de l'air chaud fourni par l'intermédiaire d'une soufflante d'air chaud incorporée au noyau de l'article.

5. Procédé, selon l'une quelconque des revendications 1 à 4, caractérisé par l'étape consistant à recouvrir l'article d'un moyen d'isolation thermique pendant la phase d'imprégnation par le liquide chaud.

6. Procédé, selon l'une quelconque des revendications précédentes, caractérisé en ce que le liquide chaud se rendant vers la surface de l'article est recirculé en ce sens qu'il revient vers le noyau et que la chaleur est ajoutée au fur et à mesure que le liquide est recirculé.

7. Procédé, selon l'une quelconque des revendications 1 à 6, caractérisé en ce que le noyau de l'article possède une ou plusieurs cavités dans lesquelles le liquide chaud est injecté.

8. Procédé, selon l'une quelconque des revendications précédentes, caractérisé en ce que la vapeur est initialement injectée dans le noyau, et ultérieurement de l'air chaud est injecté dans le noyau afin d'éliminer toute humidité résiduelle laissée par l'injection de vapeur.

9. Procédé, selon l'une quelconque des revendications précédentes, dans lequel le liquide chaud comprend de l'air chaud qui est injecté dans le noyau afin de préchauffer ce dernier, et de la vapeur est injectée à la suite de l'air chaud.

10. Article, par exemple un oreiller, un coussin, un matelas, un sofa, une couette, un fauteuil, un canapé, un jouet en peluche et autres réalisés en matière textile perméable qui constitue un milieu de reproduction favorable pour les acariens détriticoles (HDM) et qui possède un noyau, caractérisé en ce que l'article est adapté de manière à pouvoir être traité sous l'effet de la chaleur, par l'injection d'un liquide chaud de façon à ce qu'il imprègne la matière textile depuis le noyau vers l'extérieur, en vue de la dénaturation à intervalles réguliers de l'allergène des acariens détriticoles (HDM), ladite adaptation étant caractérisée en ce que l'article est muni d'un moyen d'accouplement intégré destiné à être branché sur un dispositif d'injection portable à liquide chaud grâce auquel le liquide utilisé pour effectuer l'opération de dénaturation peut être injecté dans le noyau de l'article de façon à ce qu'il imprègne l'article à partir du noyau situé à l'extérieur de celui-ci.

11. Article, selon la revendication 10, caractérisé en ce que l'article est creux à l'intérieur.

12. Article, selon la revendication 11, caractérisé en ce que le volume intérieur creux comprend une cavité unique ou une pluralité de cavités.

13. Article, par exemple un oreiller, un coussin, un matelas, un sofa, une couette, un fauteuil, un canapé, et autres réalisés en matière textile perméable qui constitue un milieu de reproduction favorable pour les acariens détriticoles (HDM) et lequel article possède un noyau, caractérisé en ce que l'article est adapté de manière à pouvoir être traité sous l'effet de la chaleur en obligeant du liquide chaud à couler depuis le noyau de façon à ce qu'il s'infiltre à partir du noyau vers l'extérieur jusqu'à la surface de l'article, en vue de la dénaturation à intervalles réguliers de l'allergène des acariens détriticoles (HDM) dans la matière textile, ladite adaptation tenant compte du fait que l'article est muni d'une soufflante d'air chaud interne intégrée qu'il est possible de mettre en circuit et hors circuit.

14. Appareil utilisé pour la dénaturation de l'allergène des acariens détriticoles (HDM) dans des articles tridimensionnels comme les oreillers, les rideaux, les matelas, les sofas, les couettes, les fauteuils, les canapés et autres qui sont réalisés en matière textile perméable, et qui constituent des milieux de reproduction favorables pour les acariens détriticoles (HDM), comprenant un moyen destiné à produire un flux de liquide chauffé, et caractérisé par une lance adaptée de façon à pouvoir pénétrer dans le noyau de l'article en vue de la diffusion de liquide chauffé dans le noyau de l'article pour qu'il puisse chauffer l'article en l'imprégnant depuis le noyau jusqu'à l'extérieur de l'article, et caractérisé en ce que la lance a été conçue pour avoir un premier tube de lance grâce auquel il est possible d'injecter de la vapeur dans le noyau de l'article, et un deuxième tube de lance grâce auquel il est possible d'injecter de l'air chaud dans le noyau de l'article, et dans lequel le premier tube de lance est positionné à l'intérieur du deuxième tube de lance.

15. Appareil, selon la revendication 14, caractérisé en ce que ledit moyen servant à fournir un flux de liquide chauffé comprend un élément de réchauffeur de process, et un agencement destiné à obliger le liquide à couler sous pression au-dessus de l'élément de réchauffeur au moment où le liquide se rend vers la lance.

16. Appareil, selon la revendication 15, comprenant un interrupteur sensible à la pression qui intervient pour disjoncter l'élément du réchauffeur au cas où il y aurait une chute de la pression du liquide passant à travers l'élément du réchauffeur.

17. Appareil destiné à effectuer la méthode indiquée à la revendication 1, caractérisé par un échangeur de chaleur fonctionnant à vapeur, un moyen pour faire passer l'air à travers l'échangeur de chaleur afin d'y être chauffé, et fournir ainsi le moyen de sécher de manière forcée la matière ou l'article, et un moyen à purge contrôlée permettant d'évacuer ladite vapeur afin d'offrir le moyen de rendre la matière ou l'article humide.
